# EUROPÄISCHE PATENTSCHRIFT

(11) **EP 1 487 773 B1**
(45) Veröffentlichungstag und Bekanntmachung des Hinweises auf die Patenterteilung: **09.01.2008**
(21) Anmeldenummer: 03711978.1
(22) Anmeldetag: 12.03.2003
(51) Int. Cl.: C07C 51/44, C07C 57/07, C07C 67/54, C07C 69/54, C07C 253/34, C07C 255/08, B01D 3/00, B01D 3/32

(54) **VERFAHREN ZUR MESSUNG DES DRUCKS IN EINER (METH)ACRYLS URE, DEREN ESTER UND/ ODER DEREN NITRILE ENTHALTENDEN GASPHASE VON REKTIFIKATIONS- UND/ ODER ABSORPTIONSKOLONNEN**
METHOD FOR THE MEASUREMENT OF THE PRESSURE IN A GAS PHASE FROM RECTIFICATION AND/OR ABSORPTION COLUMNS COMPRISING (METH)ACRYLIC ACID THE ESTERS AND/OR NITRILES THEREOF
PROCEDE DE MESURE DE LA PRESSION DANS UNE PHASE GAZEUSE CONTENANT DE L'ACIDE (METH)ACRYLIQUE, SES ESTERS ET/OU NITRILES, ISSUE DE COLONNES DE RECTIFICATION ET/OU D'ABSORPTION

(30) Priorität: 14.03.2002 DE 10211290
(43) Veröffentlichungstag der Anmeldung: 22.12.2004
(73) Patentinhaber: BASF Aktiengesellschaft, 67056 Ludwigshafen (DE)
(72) Erfinder: HAMMON, Ulrich, 68163 Mannheim (DE); SCHRÖDER, Jürgen, 67071 Ludwigshafen (DE); RISSEL, Steffen, 67281 Kirchheim (DE)
(86) Internationale Anmeldenummer: PCT/EP2003/002501
(87) Internationale Veröffentlichungsnummer: WO 2003/076382

(56) Entgegenhaltungen:
- EP-A- 1 084 740

## Beschreibung

Vorliegende Erfindung betrifft ein Verfahren zur Messung des Drucks in (Meth)acrylsäure, deren Ester und/oder deren Nitrile enthaltender Gasphase von Rektifikations- und/oder Absorptionskolonnen, in denen (Meth)acrylsäure, deren Ester und/oder deren Nitrile enthaltende Flüssigkeiten rektifikativ aufgearbeitet und/oder (Meth)acrylsäure, deren Ester und/oder deren Nitrile enthaltende Gase einer Absorption unterworfen werden, bei dem der zu messende Druck über eine offene Bohrung in der Kolonnenwand und eine sich an die offene Bohrung anschließende Leitung (z.B. eine Rohrleitung) zum Meßumformer geführt und die Leitung mit einem Gas in die Richtung zur offenen Bohrung gespült wird.

(Meth)acrylsäure steht in dieser Schrift als verkürzte Schreibweise für Acrylsäure oder Methacrylsäure. Sie, ihre Ester und/oder Nitrile sind wertvolle Ausgangsverbindungen zur Herstellung von durch radikalische Polymerisation erhältlichen Polymerisaten, die beispielsweise als Klebstoffe Verwendung finden.

(Meth)acrylsäure selbst und ihre Nitrile werden vornehmlich durch heterogen katalysierte Gasphasenoxidation und/oder Ammoxidation der entsprechenden Alkene, Alkane bzw. der korrespondierenden α,β-ethylenisch ungesättigten Aldehyde industriell hergestellt.

Dabei werden jedoch keine reinen Zielverbindungen erhalten. Vielmehr entsteht ein Produktgasgemisch, aus welchem die Zielverbindung abgetrennt werden muß. Üblicherweise wird dazu die gewünschte (Meth)acrylverbindung in einem Lösungsmittel absorbiert und nachfolgend über verschiedene Rektifikationsstufen, gegebenenfalls unter Zusatz azeotroper Schleppmittel, vom Absorptionsmittel und darin neben (Meth)acrylsäure absorbiert enthaltenen Nebenkomponenten abgetrennt. Alternativ kann das Produktgasgemisch auch fraktionierend kondensiert und das dabei gewonnene, die gewünschte (Meth)acrylverbindung enthaltende Kondensat rektifikativ aufgearbeitet werden.

Ester der (Meth)acrylsäure werden großtechnisch in der Regel durch direkte Veresterung von (Meth)acrylsäure mit Alkoholen, z.B. Alkanolen, in Gegenwart von starken Säuren und gegebenenfalls eines Schleppmittels zur Entfernung des Veresterungswassers oder durch Umesterung von (Meth)acrylsäureestern mit geeigneten Alkoholen, z.B. Alkanolen, hergestellt. Die Abtrennung des Zielesters aus dem Produktgemisch erfolgt üblicherweise ebenfalls vorwiegend rektifikativ.

Als Rektifikations- und/oder Absorptionskolonnen können für die vorgenannten Abtrennungen der (Meth)acrylverbindungen ganz generell Kolonnen mit unterschiedlichen Einbauten verwendet werden. Als solche Einbauten kommen z.B. Böden (z.B. Dual-Flow-Böden, Siebböden, Ventilböden, Thormann-Böden, Tunnelböden und/oder Glockenböden), Packungen, Raschig Ringe und/oder Pallringe in Betracht.

Nachteilig an den beschriebenen Trennverfahren ist, daß die (Meth)acrylverbindungen bei denselben vergleichsweise hohen Temperaturbelatungen ausgesetzt sind, die, selbst im Beisein von Polymerisationsinhibitoren, eine unerwünschte Polymerisation auslösen können. Die Bildung von unerwünschtem Polymerbelag, der im Extremfall die Kolonne zu verstopfen und ihre Durchlässigkeit zu mindern vermag, ist in der Regel die Folge.

Dies ist insofern von Nachteil, als sowohl die absorptive Abtrennung wie auch die rektifikative Auftrennung darauf beruhen, daß eine aufsteigende Gasphase und eine absteigende Flüssigphase im Gegenstrom zu einander und dabei nicht im Gleichgewicht miteinander stehen. Der dadurch zustandekommende Wärme- und Stoffaustausch erzeugt die gewünschte Trennwirkung. Letzteres aber nur dann, wenn die Flüssigkeits- und Gas(Dampf)-belastung so gewählt werden, daß einerseits die aufsteigende Gasphase keine nennenswerte Flüssigphase nach oben mitreißt und andererseits die Flüssigphase nicht einfach nach unten durchregnet.

Wenn nun sich bildender Polymerisatbelag in einer Kolonne deren Durchlässigkeit mindert, wird die vorstehend geschilderte Balance gestört und die Trennwirkung der Kolonne gemindert.

Ein Indikator für sich in einem bestimmten Kolonnenabschnitt ausbildenden Polymerisatbelag ist der wachsende Unterschied zwischen dem Gasdruck in der Kolonne unterhalb der Polymerisatausbildung und dem Gasdruck in der Kolonne oberhalb der Polymeriatausbildung. D.h., die zeitliche Veränderung des Unterschieds von auf verschiedenen Kolonnenhöhen gemessenen Drücken in der Gasphase der Kolonne vermögen sich ausbildenden Polymerisatbelag anzuzeigen. Selbiger kann dann durch Reinigung der Kolonne entfernt oder durch Änderung der Verdampferleistung kompensiert werden.

Die Messung von Drücken und deren Änderung in der Gasphase von Absorptions- und/oder Rektifikationskolonnen (beide Phänomene können auch überlagert auftreten) ist deshalb von erheblicher Bedeutung.

Eine Möglichkeit der Druckmessung besteht darin, den zu messenden Gasphasendruck über eine offene Bohrung in der Kolonnenwand und eine sich an die offene Bohrung anschließende Leitung (z.B. eine Rohrleitung) zu einem Meßumformer zu führen. Unter letzterem wird der eigentliche Druckmesser verstanden, mit dem der Gasdruck in ein anderes Signal, z.B. in ein elektrisches Signal, umgeformt wird.

Als Beispiel für einen Gasdruck-Meßumformer seien Kristalle (z.B. Quarz) mit piezoelektrischen Eigenschaften genannt. Bei ihnen führt eine Druckeinwirkung an der Kristalloberfläche zum Auftreten von elektrischen Ladungen, deren Größe von der Stärke der einwirkenden Kraft abhängt. Diese Tatsache ermöglicht die Ausnutzung des piezoelektrischen Effektes zur Druckmessung. Alternativ können z.B. auch Transistoren eingesetzt werden, deren Stromverstärkung und Ausgangskapazität druckabhängig sind.

Weitere Gasdruck-Meßumformer sind in Ullmanns Encyklopädie der technischen Chemie, Verlag Chemie, Weinheim, 4. Auflage, Band 5, Seiten 822 bis 832 beschrieben.

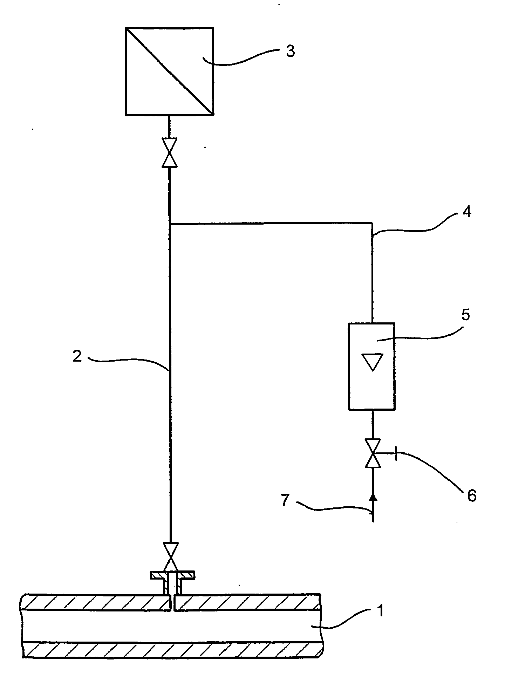

Dort wird auch empfohlen im Fall von aggressiven Gasphasen im Fall einer Druck-Messung via offene Bohrung und sich an die offene Bohrung anschließende Leitung zum Meßumformer die Druckmessung so durchzuführen, daß man die Meßleitung mit Stickstoff in der Richtung vom Meßumformer zur offenen Bohrung kontinuierlich spült. Das Spülmedium muß dabei mit höherem Druck als das zu ermittelnde Gasmedium genau und zeitlich konstant dosiert zugeführt werden (der Zusatzdruck des Spülgases ist in der Eichung berücksichtigt). Eine Druckänderung des Spülstromes führt zu Meßfehlern. Beispielsweise können zur Dosierung des Spülgases Schwebekörpermesser eingebaut werden, wenn notwendig mit selbsttätiger Regelungseinrichtung (vgl. Figur 1; 1 = Kolonne, 2 = Meßleitung, 3 = Meßumformer, 4 = Spülleitung, 5 = Schwebekörpermesser, 6 = Regulierventil, 7 = molekularer Stickstoff).

Nachteilig an einer solchen Stickstoffspülung im Fall von (Meth)acrylverbindungen enthaltenden Gasphasen ist jedoch, daß trotz der Spülung die Meßleitungen am der Kolonne zugewandten Ende im Lauf der Betriebszeit infolge von unerwünschter Polymerisatbildung verstopfen.

Aufgabe der vorliegenden Erfindung war es daher, ein verbessertes Verfahren der Druckmessung zur Verfügung zu stellen.

Aus der EP-A 856 343 und der EP-A 10 34 824 war das Problem der unerwünschten Polymerisatbildung von (Meth)acrylverbindungen in Toträumen bekannt. Als Lösung empfiehlt die EP-A 10 84 740 derartige Toträume mit einem molekularen Sauerstoff enthaltenden Gas als Gegenmaßnahme zu spülen. Derartige Toträume liegen bei vorgenannter Druckmessung mit Stickstoffspülung der Meßleitung nicht vor. Dieser Tatsache entsprechend erfolgt keine Polymerisatbildung in der Meßleitung selbst.

Als Lösung der Aufgabe wurde ein Verfahren zur Messung des Drucks in (Meth)acrylsäure, deren Ester und/oder deren Nitrile enthaltender Gasphase von Rektifikations- und/oder Absorptionskolonnen, in denen (Meth)acrylsäure, deren Ester und/oder deren Nitrile enthaltende Flüssigkeiten rektifikativ aufgearbeitet und/oder (Meth)acrylsäure, deren Ester und/oder deren Nitrile enthaltende Gase einer Absorption unterworfen werden, bei dem der zu messende Druck über eine offene Bohrung in der Kolonnenwand und eine sich an die offene Bohrung anschließende Leitung (z.B. eine Rohrleitung) zum Meßumformer geführt und die Leitung mit einem Gas in die Richtung zur offenen Bohrung gespült wird, gefunden, das dadurch gekennzeichnet ist, daß die Leitung mit einem molekularen Sauerstoff enthaltenden Gas gespült wird.

Fig. 1 zeigt eine mögliche Ausführungsvariante des erfindungsgemäßen Verfahrens, wenn man sich den molekularen Stickstoff = 7 durch ein molekularen Sauerstoff enthaltendes Spülgas ersetzt denkt.

Erfindungsgemäß bevorzugt sind als molekularen Sauerstoff enthaltende Spülgase solche, deren Gehalt an molekularem Sauerstoff 1 bis 50 Vol.-%, besonders bevorzugt 4 bis 21 Vol.-% beträgt. Selbstredend könnte aber auch reiner molekularer Sauerstoff verwendet werden.

Bei der rektifikativen Auftrennung von (Meth)acrylverbindungen enthaltenden Flüssigkeiten bzw. bei der Absorption von (Meth)acrylverbindungen enthaltenden Gasen mit einem Flammpunkt (bestimmt nach DIN EN 57) von ≤ 50°C ist ein Gehalt des verwendeten Spülgases an molekularem Sauerstoff von 4 bis 10 Vol.-% ganz besonders bevorzugt.

Der Begriff (Meth)acrylsäureester umfaßt in dieser Anmeldung insbesondere die Ester der (Meth)acrylsäure mit C₁- bis C₁₂-, bevorzugt mit C₁- bis C₈-Alkanolen und/oder Alkandiolen. Das sind vor allem Methylacrylat, Methylmethacrylat, Ethylacrylat, Ethylmethacrylat, n-Butylacrylat, tert.-Butylacrylat, tert. Butylmethacrylat sowie 2-Ethylhexylacrylat, aber auch die Ester des Dimethylaminoethanols.

Die nach dem erfindungsgemäßen Verfahren rektifikativ behandelten, (Meth)acrylsäure, deren Ester und/oder deren Nitrile enthaltenden Flüssigkeiten können ≥ 20 Gew.-%, oder ≥ 40 Gew.-%, oder oder ≥ 60 Gew.-%, oder ≥ 80 Gew.-%, oder ≥ 90 Gew.-%, oder ≥ 95 Gew.-%, oder ≥ 99 Gew.-% der (Meth)acrylverbindungen enthalten.

Die Rektifikation kann unter Überdruck, Normaldruck oder reduziertem Druck durchgeführt werden. Selbstredend werden die Rektifikations- und/oder Absorptionskolonnen beim erfindungsgemäßen Verfahren normalerweise polymerisiationsinhibiert betrieben. In der Regel werden dazu die Polymerisationsinhibitoren in den Rücklauf gegeben. Als Polymerisationsinhibitoren kommen dabei alle in an sich bekannter Weise verwendbaren Inhibitoren in Betracht. Beispielhaft genannt seien Phenothiazin, Hydrochinon oder Hydrochinonmonomethylether. Als weitere Stabilisierungsmaßnahme kann zusätzlich ein molekularen Sauerstoff enthaltendes Gas, z.B. Luft, durch die Kolonne geführt werden.

### Beispiele

### Beispiel 1

In einer Bodenkolonne (Material: ein Edelstahl mit der Werkstoffnummer 1.4571 entsprechend der Norm DIN EN 10020) mit 3,8 m Durchmesser und einer Länge von 32 m wurden Druckmessstutzen mit einer Nennweite (Innendurchmesser der Bohröffnung) von 25 mm und einer Länge von 400 mm an der Kolonnenwand bündig.angeschweisst.

Vierzehn dieser Messstutzen wurden entlang der Kolonnenwand vertikal derart angebracht, daß die Bohröffnung eines Stutzens zwischen zwei Böden der Trennkolonne lag. Der Abstand zwischen zwei aufeinanderfolgenden, der insgesamt 45, Dual-Flow-Böden (Material: ein Edelstahl mit der Werkstoffnummer 1.4571 entsprechend der Norm DIN EN 10020) betrug über die gesamte Kolonne homogen 400 mm. Die Oberkante der Bohröffnung wurde jeweils 10 cm unterhalb des Bodenblechs angebracht.

Jeweils zwei der Messstutzen wurden mit einer Rohrleitung mit einer Nennweite von 12 mm mit einem Differenzdruckmeßumformer verbunden.

In der Bodenkolonne wurde aus der wie nachfolgend beschrieben zusammengesetzten Flüssigkeit (Zuflußmenge = 114 Tonnen/h über die Zulaufleitung der Kolonne) Acrylsäure rektifikativ abgetrennt.

Die Flüssigkeit enthielt:

| | |
|---|---|
| 17 Gew.-% | Acrylsäure, |
| 0,02 Gew.-% | Wasser, |
| 0,0015 Gew.-% | Acrolein, |
| 0,0015 Gew.-% | Allylacrylat, |
| 0,01 Gew.-% | Furfural, |
| 0,027 Gew.-% | Essigsäure, |
| 0,2 Gew.-% | Benzaldehyd, |
| 0,003 Gew.-% | Propionsäure, |
| 0,032 Gew.-% | Maleinsäureanhydrid, |
| 58 Gew.-% | Diphyl, |
| 17,0 Gew.-% | Dimethylphthalat, |
| 3 Gew.-% | Acryloylpropionsäure und |
| 0,02 Gew.-% | Phenothiazin. |

37 Böden befanden sich oberhalb der Zulaufstelle und 8 Böden befanden sich unterhalb der Zulaufstelle der die Acrylsäure enthaltenden Flüssigkeit.

Die Dual-Flow-Böden oberhalb des Zulaufs wiesen Bohrlöcher des Durchmessers 25 mm und die Dual-Flow-Böden unterhalb des Zulaufs wiesen Bohrlöcher des Durchmessers 50 mm (jeweils innen gemessen) auf. Die Acrylsäure haltige Flüssigkeit wurde in eine 99,6 Gew.-%ige Acrylsäure, eine Gemisch aus den leichter als Acrylsäure siedenden Komponenten, das weniger als 96 Gew.-% Acrylsäure enthielt, und ein Gemisch aus schwerer als Acrylsäure siedenden Komponenten, das weniger als 0,5 Gew.-% Acrylsäure enthielt, aufgetrennt. Die Temperatur am Kopf der Kolonne betrug 80°C, der Druck am Kolonnenkopf betrug 105 mbar und das Rücklaufverhältnis lag bei 1,3. Die Temperatur am Sumpf der Kolonne betrug 193°C und der Druck am Kolonnensumpf lag bei 230 mbar. Der Rücklauf der Kolonne wurde mit Phenothiazin so stabilisiert, daß die über Seitenabzug (auf Boden 35, von unten gerechnet) entnommene 99,6 gew.-%ige Acrylsäure 250 gew.-ppm PTZ enthielt. Das PTZ wurde in solchermaßen abgetrennter Acrylsäure gelöst (als 1,5 gew.-%ige Lösung) zugesetzt. Zusätzlich wurden zur Stabilisierung in den unteren Teil der Rektifikationskolonne 400 000 Nl/h Luft eingeleitet.

Insgesamt (einschließlich der Spülluft in die Rohrleitungen) wurden der Rektifikationskolonne somit 84294 N1/h molekularer Sauerstoff zugeführt.

Innerhalb einer Laufzeit von 35 Tagen mußte lediglich die Bohröffnung eines der 14 Meßstutzen von Polymerisat gereinigt werden.

### Beispiel 2

Es wurde wie in Beispiel 1 verfahren. Anstelle von Luft wurde jedoch eine Mischung aus 7 Vol.-% Sauerstoff und 93 Vol.-% Stickstoff als Spülgas in gleicher Menge verwendet. Ferner wurden in den unteren Teil der Rektifikationskolonne 410 000 N1/h Luft eingeleitet. Insgesamt wurden der Rektifikationskolonne somit 86200 N1/h molekularer Sauerstoff zugeführt.

Innerhalb einer Laufzeit von 35 Tagen mussten die Bohröffnungen von 5 Messstutzen von Polymerisat gereinigt werden.

### Vergleichsbeispiel 1

Es wurde wie in Beispiel 1 verfahren. Anstelle mit Luft wurde jedoch mit molekularem Stickstoff gespült. Zusätzlich wurden zur Stabilisierung in den unteren Teil der Rektifikationskolonne 420 000 N1/h Luft eingeleitet.

Insgesamt wurden der Rektifikaitonskolonne somit 88200 N1/h molekularer Sauerstoff zugeführt.

Innerhalb einer Laufzeit von 35 Tagen mussten die Bohröffnungen aller 14 Messstutzen mehrmals von Polymerisat gereinigt werden.

### Beispiel 3

In einer Rektifikationskolonne (Material: ein Edelstahl mit der Werkstoffnummer 1.4541 entsprechend der Norm DIN EN 10020) wurde ein Produktgemisch getrennt, das die nachfolgen Bestandteile enthielt:

| | |
|---|---|
| 2,5 Gew.-% | Methanol, |
| 0,004 Gew.-% | Methacrylsäure, |
| 96,3 Gew.-% | Methylmethacrylat, |
| 0,5 Gew.-% | Methylacetat, |
| 0,06 Gew.-% | Methylpropionat |
| 1,4 Gew.-% | Wasser |
| 0,01 Gew.-% | Hydrochinon |

In der Rektifikationskolonne wurde das Produktgemisch aufgetrennt, in ein Leichtsiedergemisch mit weniger als 23 Gew.-% Methylmethacrylat und ein Schwersiedergemisch mit mehr als 99 Gew.-% Methylmethacrylat. Als trennwirksame Einbauten enthielt die Kolonne 60 Dual-Flow-Böden (mit Bohrlöchern des Durchmessers 15 mm, Material: ein Edelstahl mit der Werkstoffnummer 1,4541 entsprechend der Norm DIN EN 10020). Der Zulauf war auf Boden 50 (von unten gerechnet).

Die Temperatur am Kolonnenkopf betrug 101°C, der Druck betrug 930 mbar und das Rücklaufverhältnis betrug 2,2. Die Temperatur am Kolonnensumpf betrug 105°C und der Druck betrug 1110 mbar.

Die Rektifikationskolonne enthielt 8 offene Bohrungen in der Kolonnenwand an die sich Leitungen zum jeweiligen Druckmeßumformer anschlossen. Jede Zuleitung zu den Druckmeßumformern wurde mit 100 Nl/h eines Gemisches aus 92 Vol.-% Stickstoff und 8 Vol.-% Sauerstoff gespült. Zusätzlich wurden zur Stabilisierung in den unteren Teil der Rektifikationskolonne 400 Nl/h Luft eingeleitet. Im übrigen enthielt der Rücklauf der Rektifikationskolonne 100 Gew.-ppm Hydrochinon.

Die Zufuhr an molekularem Sauerstoff betrug so insgesamt 148 Nl/h.

Nach einer Laufzeit von 180 Tagen waren noch alle Druckmeßeinrichtungen funktionsfähig.

### Vergleichsbeispiel 2

Es wurde wie in Beispiel 3 verfahren. Die Spülung der Zuleitungen zu den Druckmeßumformern wurde jedoch mit 100 N1/h Stickstoff gespült. Außerdem wurden in den unteren Teil der Rektifikaitonskolonne 800 N1/h Luft eingeleitet.

Die Zufuhr an molekularem Sauerstoff betrug so insgesamt 168 N1/h.

Nach einer Laufzeit von 30 Tagen waren 6 der 8 offenen Bohrungen in der Kolonnenwand zupolymerisiert.

## Patentansprüche

1. Verfahren zur Messung des Drucks in (Meth)acrylsäure, deren Ester und/oder deren Nitrile enthaltender Gasphase von Rektifikations- und/oder Absorptionskolonnen, in denen (Meth)acrylsäure, deren Ester und/oder Nitrile enthaltende Flüssigkeiten rektifikativ aufgearbeitet und/oder (Meth)acrylsäure, deren Ester und/oder deren Nitrile enthaltende Gase einer Absorption unterworfen werden, bei dem der zu messende Druck über eine offene Bohrung in der Kolonnenwand und eine sich an die offene Bohrung anschließende Leitung zum Meßumformer geführt und die Leitung mit einem Gas in die Richtung zur offenen Bohrung gespült wird, **dadurch gekennzeichnet, daß** die Leitung mit einem molekularen Sauerstoff enthaltenden Gas gespült wird.

2. Verfahren nach Anspruch 1, **dadurch gekennzeichnet, daß** die Leitung mit Luft gespült wird.

## Claims

1. A process for measuring the pressure in a gas phase comprising (meth)acrylic acid, the esters and/or nitriles thereof of a rectification and/or absorption column where liquids comprising (meth)acrylic acid, the esters and/or nitriles thereof are worked up rectificatively and/or gases comprising (meth)acrylic acid, the esters and/or nitriles thereof are subjected to an absorption, said pressure to be measured being transferred to a transducer via an open drillhole in the column wall and a line which is connected to the open drillhole and is purged with a gas in the direction toward the open drillhole, which comprises purging the line with a molecular oxygen-comprising gas.

2. The process according to claim 1, wherein the line is purged with air.

## Revendications

1. Procédé de mesure de la pression dans une phase gazeuse contenant de l'acide (méth)acrylique, ses esters et/ou nitriles, issue de colonnes de rectification et/ou d'absorption dans lesquelles les liquides contenant de l'acide (méth)acrylique, ses esters et/ou nitriles sont traités par rectification et/ou les gaz contenant l'acide (méth)acrylique, ses esters et/ou nitriles sont soumis à une absorption, dans lequel la pression à mesurer est guidée par l'intermédiaire d'un alésage ouvert dans la paroi de colonne et une conduite adjacente à l'alésage ouvert vers le transducteur de mesure et la conduite est rincée avec un gaz dans la direction de l'alésage ouvert, **caractérisé en ce que** la conduite est rincée avec un gaz contenant de l'oxygène moléculaire.

2. Procédé selon la revendication 1, **caractérisé en ce que** la conduite est rincée avec de l'air.
